(19) Europäisches Patentamt
European Patent Office
Office européen des brevets

(11) EP 0 966 414 B1

(12) **EUROPEAN PATENT SPECIFICATION**

(45) Date of publication and mention of the grant of the patent:
**04.06.2003 Bulletin 2003/23**

(21) Application number: **98910833.7**

(22) Date of filing: **06.03.1998**

(51) Int Cl.[7]: **C07B 61/02**, C07D 519/00, A61K 49/00, G01N 24/12, G01R 33/62

(86) International application number:
**PCT/GB98/00672**

(87) International publication number:
**WO 98/039277 (11.09.1998 Gazette 1998/36)**

(54) **TRIARYLMETHYL FREE RADICALS AS IMAGE ENHANCING AGENTS**
TRIARYLMETHYLFREIE RADIKALE ALS BILDVERBESSERUNGSMITTEL
RADICAUX TRIARYLMETHYLE LIBRES UTILISES COMME AGENTS D'AMELIORATION D'IMAGE

(84) Designated Contracting States:
**DE ES FR GB IT**

(30) Priority: **06.03.1997 GB 9704669**
**25.06.1997 US 882147**

(43) Date of publication of application:
**29.12.1999 Bulletin 1999/52**

(73) Proprietor: **Amersham Health AS**
**0485 Oslo (NO)**

(72) Inventors:
- **THANING, Mikkel**
**S-205 12 Malmo (SE)**
- **PETTERSSON, Göran**
**20512 Malmö (SE)**

(74) Representative: **Rollins, Anthony John et al**
**Amersham plc**
**Amersham Place**
**Little Chalfont, Bucks. HP7 9NA (GB)**

(56) References cited:
**WO-A-96/39367**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to novel triarylmethyl free radicals and their use as image enhancing agents in magnetic resonance imaging (MRI), in particular to their use in electron spin resonance enhanced magnetic resonance imaging (OMRI) of a sample (for example a human or animal body) for determining the oxygen concentration of said sample.

MRI is a diagnostic technique that has become particularly attractive to physicians as it is non-invasive and does not involve exposing the patient under study to potentially harmful radiation (eg. X-rays) The technique, however, suffers from *inter alia* the problem of achieving effective contrast in the magnetic resonance (MR) images between tissue types having the same or closely similar imaging parameters.

Electron spin resonance enhanced MRI, referred to herein as OMRI (Overhauser MRI) but also referred to in earlier publications as ESREMRI or PEDRI, is a particular form of MRI in which enhancement of the magnetic resonance signals from which the images are generated is achieved by virtue of the dynamic nuclear polarization (the Overhauser effect) that occurs on VHF stimulation of an esr transition in a paramagnetic material, generally a persistent free radical, in the subject under study. Magnetic resonance signal enhancement may be by a factor of a hundred or more thus allowing OMRI images to be generated rapidly and with relatively low primary magnetic fields.

OMRI techniques have been described by several authors, notably Leunbach, Lurie, Ettinger, Grücker, Ehnholm and Sepponen, for example in EP-A-296833, EP-A-361551, WO-A-90/13047, J. Mag. Reson. 76:366-370(1988), EP-A-302742, SMRM 9:619(1990), SMRM 6:24(1987), SMRM 7:1094(1988), SMRM 8:329(1989), US-A-4719425, SMRM 8:816(1989), Mag. Reson. Med. 14 : 140-147 (1990), SMRM 9:617(1990), SMRM 9:612(1990), SMRM 9:121 (1990), GB-A-2227095, DE-A-4042212 and GB-A-2220269.

In the basic OMRI technique, the imaging sequence involves initially irradiating a subject placed in a uniform magnetic field (the primary field $B_o$) with radiation, usually VHF radiation, of a frequency selected to excite a narrow linewidth esr transition in a paramagnetic enhancement agent (hereinafter "an OMRI contrast agent") which is in or has been administered to the subject. Dynamic nuclear polarization results in an increase in the population difference between the excited and ground nuclear spin states of the imaging nuclei, i.e. those nuclei, generally protons, which are responsible for the magnetic resonance signals. Since MR signal intensity is proportional to this population difference, the subsequent stages of each imaging sequence, performed essentially as in conventional MRI techniques, result in larger amplitude MR signals being detected and more effective contrast.

A number of oxygen free radicals that is to say radicals where the unpaired electron or electrons are associated with the oxygen atom have been proposed as OMRI contrast agents including for example nitroxide stable free radicals, chloranil semiquinone radical and Fremy's salt (US-A-4984573) and deuterated stable free radicals, in particular deuterated nitroxide stable free radicals (WO-A-90/00904).

In WO-A-91/12024, Nycomed Innovation AB proposed persistant carbon free radicals, i.e. radicals (eg. triarylmethyl radicals) in which the unpaired electron(s) are primarily associated with carbon atoms, for use as OMRI contrast agents.

In WO-A-96/39367, Nycomed Imaging AS proposed various sulphur-based triarylmethyl radicals for use as OMRI contrast agents.

In any OMRI experiment under ambient conditions, paramagnetic oxygen will have a finite effect on the spin system present. Generally speaking, this may be dismissed as a secondary effect when compared to the primary interaction of the radical electron spin and the nuclear spin system. Nonetheless, it has been proposed that this effect may be used to determine oxygen concentration within a sample. Research has concentrated particularly on the use of nitroxide spin labels; radicals which suffer the inherent disadvantage of having broad linewidth esr resonances and therefore low sensitivity to the effects of oxygen. Thus, to date, the effect of oxygen has been recognised only in a qualitative sense and any attempt to attach a quantitative significance to the oxygen effect has failed. Moreover, in general, non-invasive techniques for oxygen determination have been slow to develop and typically are not suited to the study of tissues lying deep beneath the surface of a sample.

For example, Grücker et al (MRM, 34 : 219-225(1995)) reported a method for calculating oxygen concentration by measuring the Overhauser effect attributable to a nitroxide radical and relating the non-linear effect of oxygen on the Overhauser Factor to its concentration. This involved taking two images, one on-resonance and one off-resonance, and using a first order approximation to arrive at the oxygen concentration. However, Grücker observed that the correlation between actual and calculated oxygen concentration was poor and therefore that the method was inherently inaccurate. This was attributed to the large number of parameters involved in the calculation.

Ehnholm (US-A-5289125) proposed an OMRI technique in which signals from a paramagnetic material were detected under at least two different sets of operating parameters whereby to generate images of various physical, chemical or biological parameters. While oxygen tension was one of several such parameters, Ehnholm did not demonstrate the use of the technique to quantitate dissolved oxygen.

It has now been found that certain novel sulphur based triarylmethyl radicals have advantageous properties

for example an improved metabolism pattern which makes them particularly suitable for use as QMRI contrast agents.

Viewed from one aspect the present invention provides a radical compound of formula I

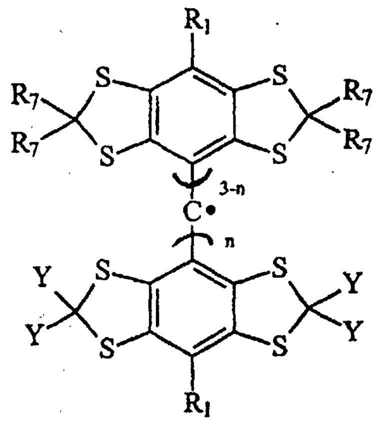

(I)

(wherein:

each $R^1$ which may be the same or different represents a hydrogen atom or group of formula -M or -XM;
each X which may be the same or different represents an oxygen or sulphur atom or a group CO or $S(O)_m$ (where m is 1 to 3);
M represents a water solubilising group;
each $R^7$ which may be the same or different represents a hydrogen atom, or a hydrocarbon group such as an alkyl, hydroxyalkyl, alkoxyalkyl, alkoxycarbonyl, or carbamoyl group, or a water solubilising group M or two groups $R^7$ together with the atom to which they are bound represent a carbonyl group or a 5 to 8 membered cycloalkylidene, mono- or di-oxacycloalkylidene, mono- or di-azacycloalkylidene or mono- or di-thiacycloalkylidene group (preferably however in any spiro structure the ring linking atom will be bonded to no more than three heteroatoms) and $R^7$ where it is other than hydrogen, is optionally substituted by a hydroxyl group, an optionally alkoxylated, optionally hydroxylated acyloxy or alkyl group or a water solubilising group M;
n denotes 1, 2 or 3; and
each group Y denotes $CH_2CH_2OR_{22}$, wherein $R_{22}$ is H or $C_{1-6}$-alkyl, (e.g. a $CH_2CH_2OH$ group), preferably H or $^tBu$, or a deuterated analog thereof,

or a deuterated analog, precursor or salt thereof.

This definition covers radical precursors which may conveniently undergo a radical generation step shortly before administration or even in situ to produce the desired free radical. Radical precursors and radical generation steps are well-known to those skilled in the art.

Preferred radical compounds according to the invention include those of formula I wherein n is at least two and more preferably is three.

In the radical compounds according to the invention, the solubilising groups M may be any of t solubilising groups conventionally used in diagnostic and pharmaceutical products. Particularly preferred solubilizing groups M include optionally hydroxylated, optionally alkoxylated alkyl or oxo-alkyl groups and groups of formulae $R^5$, $COOR^5$, $OCOR^5$, CHO, CN, $CH_2S(O)R^5$ $CONR^5_2$, $NR^5COR^5$, $NR^5_2$ $SO_2NR^5_2$ $OR^5$, $PO_3^{2-}$ , $SOR^5$, $SO_2R^5$, $SO_3M^1$ $COOM^1$ (where $R^5$ represents a hydrogen atom or an optionally hydroxylated, optionally aminated, optionally alkoxylated, optionally carboxylated alkyl, oxo-alkyl, alkenyl or alkaryl group and $M^1$ is one equivalent of a physiologically tolerable cation, for example an alkali or alkaline earth metal cation, an ammonium ion or an organic amine cation, for example a meglumine ion), $-(O(CH_2)_n)_mOR^5$ (where n is an integer having a value of from 1 to 3 and m is an integer having a value of from 1 to 5), $-CX(CHR^5)_nX$ or $CH_2R^8$ (where $R^8$ is a hydrophilic $R^5$ group) or $SR^{10}$ or $SO_2R^{10}$ where $R^{10}$ is a group $R^5$ or an alkyl group optionally substituted by one or more, especially two or three groups $COOR^5$, $OCOR^5$, CHO, CN, $CONR^5_2$, $NR^5COR^5$, $NR^5_2$, $SO_2NR^5_2$, $OR^5$, $PO_3^{2-}$, $SOR^5$, $SO_2R^5$, $SO_3M^1$, $COOM^1$, or $-(O(CH_2)_n)_mOR^5$.

Especially preferred as solubilizing groups M are groups of formula $C(H)_{3-n}(CH_2OH)_n$, $R^9$, $COR^9$, $SR^9$, $SOR^9$, $SO_2R^9$, CON $(R^9)_2$, $NR^9_2$, $NHR^9$ and $CONHR^9$ [where $R^9$ may represent a hydroxylated alkyl group such as a group

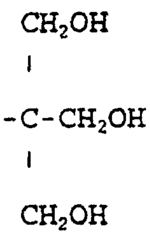 , 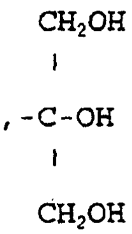 $-CH_2CH_2OH$, $-CH_2OH$

$-CH_2CHOHCH_2OH$, 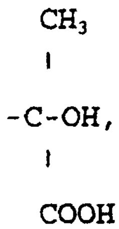 $-O-CH_2CH_2OH$, $-CH_2OCH_2CH_2OH$,

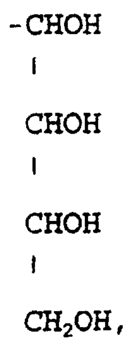 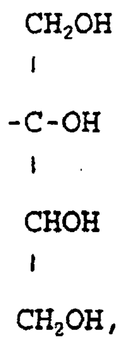

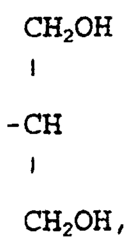 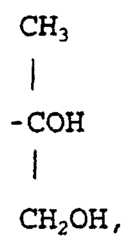 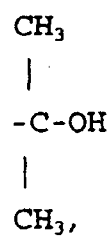 or 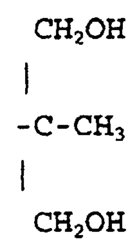

(although any $R^9$ group attached to a sulphur, nitrogen or oxygen atom is preferably not hydroxylated at the α carbon)], and groups of formula $SO_2R^{12}$ or $SR^{12}$ where $R^{12}$ is a group $CH_2COOR^{13}$, $CH(COOR^{13})_2$, $CH_2CONHR^9$, $CH_2CONR^9{}_2$, $CR^5(COOR^{13})_2$, $CH(CN)CO_2R^{13}$, $(CH_2)_nSO_3^-M^1$, $(CH_2)_nCOR^{13}$, $CH(COR^9)CH_2COR^9$ and $CH(R^5)COR^9$ where n, $M^1$ and $R^5$ are as hereinbefore defined and $R^{13}$ is a hydrogen atom, an alkyl group or a group $M^1$ or $R^9$.

In the radical compounds according to the invention, X is preferably selected from oxygen or sulphur atoms or $SO_2$ groups. Preferably two and especially preferably all X groups are identical, especially preferably they are all sulphur atoms.

In the radical compounds according to the invention preferred identities for the group $R^1$ may be selected from the group consisting of -H, $-SCH_2COO^-Na^+$, $-SO_2R^2$, $-SR^2$, $-SCH_2COOCH_2CH_3$, $-SO_2C(R^2)_2CH_2CHOHCH_2OH$, $-SO_2NR^2{}_2$, $-SO_2CH_2CON(R^2)_2$, $-C-(CH_2CH_2OH)_3$, $-SO_2-C(H)(COOCH_2CH_3)_2$, $-CH_2CON(CH_2CH_2OH)_2$, -COOH, $-CO_2Me$, $CO_2Et$, $-CO_2M^1$

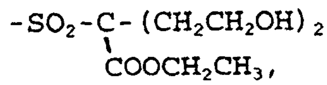

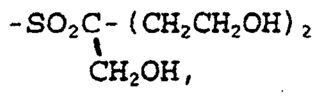

(where $M^1$ is as hereinbefore defined and $R^2$ is H or optionally hydroxylated alkyl eg. $CH_2CH_2OH$, $CH_2CHOHCH_2OH$, $CH_3$, $CH_2CH_3$, $CH_2(CHOH)_4CH_2OH$) or deuterated analogues thereof.

The most preferred compounds according to the invention are:

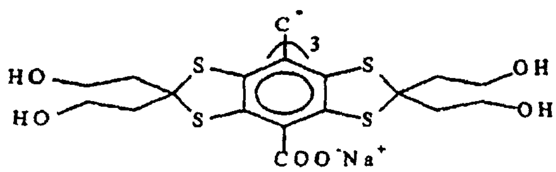

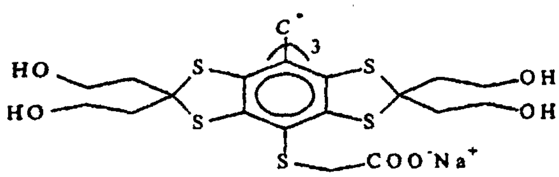

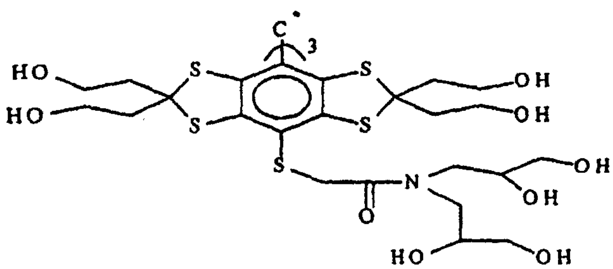

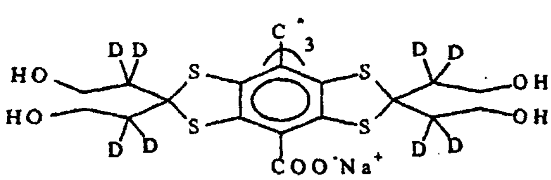

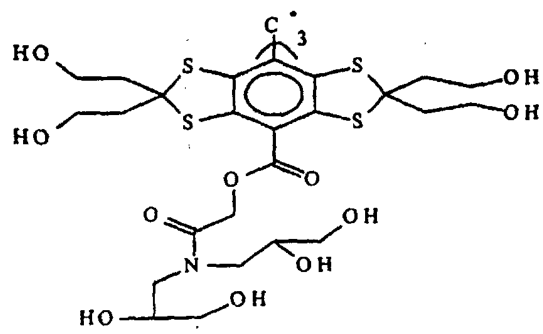

Compounds according to the invention have been found to have an improved metabolism pattern, namely a half-life which is typically greater than 30 minutes when a dose of 0.05 mmol/kg is injected into a rat (where the half-life is defined as the time from injection until the radical concentration in the blood equates with the metabolite concentration).

Further aspects of the invention provide the use of a radical compound according to the invention for the manufacture of a contrast medium for use in OMRI and a method of magnetic resonance investigation of a sample, said method comprising introducing into said sample a radical compound according to the invention, exposing said sample to a first radiation of a frequency selected to excite electron spin transitions in said radical, exposing said sample to a second radiation of a frequency selected to excite nuclear spin transitions in selected nuclei (eg. protons) in said sample, detecting free induction decay signals from said sample, and, optionally, generating an image or dynamic flow data from said detected signals.

Viewed from a still further aspect, the invention provides a magnetic resonance imaging contrast medium composition comprising a radical compound according to the invention together with at least one pharmacologically acceptable carrier or excipient.

For *in vivo* imaging, the radical compound should preferably be a physiologically tolerable radical, or one presented in a physiologically tolerable, e.g. encapsulated, form.

For the method according to the invention the preferred radicals are those which have relatively few transitions, e.g. less than 15, preferably less than 10, in their ESR spectra and radicals having narrow linewidth ESR transitions, e.g. up to 500 mG, preferably less than 150 mG, especially less than 60 mG and particularly less than 25mG, are especially preferred. (The linewidths referred to are conveniently the intrinsic linewidths (full width at half maximum in the absorption spectrum) under ambient conditions).

Whilst low numbers of ESR transition lines are generally preferred to obtain more effective coupling of the ESR and NMR transitions, good coupling (and therefore enhancement of the MR signal) may also be achieved with radicals showing a large number of ESR transitions.

Where the radicals have a multiplicity of ESR transitions, the hyperfine splitting constant is preferably very small. In this connection radicals having as few as possible non-zero spin nuclei, positioned as far away as possible from the paramagnetic centre are thus especially preferred.

The radical compounds according to the invention may be coupled to further molecules for example to lipophilic moieties such as long chain fatty acids or to macromolecules, such as polymers, proteins, polysaccharides (e.g. dextrans), polypeptides and polyethyleneimines. The macromolecule may be a tissue-specific biomolecule such as an antibody or a backbone polymer such as polylysine capable of carrying a number of independent radical groups which may itself be attached to a further macromolecule. Coupling to lipophilic molecules or substitution of the radical with lipophilic groups is particularly useful since it may enhance the relaxivity of the radicals in certain systems such as blood. Such lipophilic and macromolecular derivatives of the radical compound of formula I and salts thereof form a further aspect of the present invention.

The linkage of a compound according to the invention to the further molecule may be effected by any of the conventional methods such as the carbodiimide method, the mixed anhydride procedure of Krejcarek et al. (see Biochemical and Biophysical Research Communications 77: 581 (1977)), the cyclic anhydride method of Hnatowich et al. (see Science 220: 613 (1983) and elsewhere), the backbone conjugation techniques of Meares et al. (see Anal. Biochem. 142: 68 (1984) and elsewhere) and Schering (see EP-A-331616 (Deutsch/Schering) for example) and by the use of linker molecules as described for in US-A-5208324 (Klaveness/Nycomed).

In view of their beneficial properties, one further aspect of the present invention is the use of the radical compounds of the invention as conventional OMRI contrast agents, as ESR contrast agents or as ESR spin labels in ESR imaging or in magnetometry.

The radical compounds according to the invention have been found to be useful in providing a non-invasive method based on OMRI for determining the oxygen concentration of a sample (hereinafter the OXI method) which involves manipulation of the Overhauser effect attributable to the radical compounds of the invention. More specifically, the method is based on observing and manipulating the varying enhancement of a proton signal due to the changed saturation characteristics of the radical in the presence of oxygen.

Viewed from a yet further aspect the present invention provides a method of determining oxygen concentration in a sample, for example a human or non-human, preferably mammalian, subject, said method comprising the following steps:

introducing into said sample an effective amount of a physiologically tolerable radical compound according to the invention, preferably a radical having an esr transition with a linewidth (measured in water at 37°C) of less than 400mG, more preferably less than 150mG;
irradiating said sample with radiation of an amplitude (i.e. power) and frequency selected to stimulate an electron spin resonance transition of said radical ;
detecting electron spin resonance enhanced magnetic resonance signals from said sample under at least first, second and third conditions, whereby under said first and second conditions said radiation is of a first frequency, under said third conditions said radiation is of a second frequency different from said first frequency, under said first, second and third conditions said radiation is of a first, second and third amplitude, said first and second amplitudes at least being different from each other; and
manipulating said detected signals whereby to determine oxygen concentration in said sample.

In a preferred embodiment, the OXI method comprises:

(a) introducing a radical compound according to the invention, e.g. parenterally, for example by injection into body tissue or into the vasculature;
(b) generating a first OMRI image of said sample at VHF power $P_A$, irradiation period $T_{VHF1}$ and on-resonance ($\Delta H=O$) (i.e. where the frequency of the radiation is selected to be the resonance frequency of the esr transition);
(c) generating a second OMRI image of said sample at a second VHF power $P_B$, irradiation time $T_{VHF1}$ and on-resonance ($\Delta H=O$) ;
(d) generating a third OMRI image of said sample at VHF power $P_c$ (eg equal to $P_A$ or $P_B$), irradiation time $T_{VHF1}$ and off-resonance ($\Delta H\neq O$, for example 100-200mG);
(e) manipulating the images obtained in steps (b) to (d) and calibrating using parameters determined ex *vivo* to provide an oxygen image of said sample.

In an especially preferred embodiment, a fourth and fifth OMRI image are additionally generated in the imaging sequence. The conditions for the fourth image are identical to the first image but the VHF irradiation time $T_{VHE2}$ is different (for example twice as long, i.e. $T_{VHF2}=2T_{VHF1}$) and the fifth image is obtained without VHF irradiation, eg. is a native image of intensity $I_o$, generated by conventional MRI with a repetition time $T_R=T_{VHF}$.

In a further embodiment, a native image (i.e. one obtained by conventional MRI) of the sample (e.g. body) may be generated to provide structural (e.g. anatomical) information upon which the oxygen image may be superimposed. In this way, precise location of, for example, an oxygen deficient tumour will be possible.

Accurate measurement of the level of oxygen in bodily tissues is an invaluable aid to the clinician and the OXI method has a variety of end uses. For example, knowledge of the concentration of oxygen dissolved in blood can be used (through known rate constants) to calculate the concentration of oxygen associated with haemoglobin. This is a useful parameter which is presently measured either by undesirable invasive techniques or using the BOLD MR imaging technique which involves high field imaging to determine the effect of oxygen on paramagnetic iron but which has the disadvantage that to determine blood oxygen concentration the volume of blood in which the measurement was made needs to be known.

Other uses of the OXI method will be readily apparent to the skilled person and include oxygen imaging (e.g. mapping) of, for example, the heart and arteries and of malignant tumours, for example in the brain, breast, lung, lymphoid tissues and superficial areas of the liver. In the case of oxygen imaging of tumours, success in treatment of malignant tumours by radiotherapy may be reflected in the level of oxygen in the tissue (typically an oxygen concentration of less than 0.01mM will indicate that the tissue is necrotic and thus that treatment is likely to be ineffective).

It will also be apparent that the OXI method will be useful in cardiology, surgery and intensive care where levels of oxygen and even perfusion can be non-invasively assessed in almost any tissue.

The manipulation of the detected MR signals in the OXI method will generally be to generate an image data set (i.e. a data set from which an image may be generated) indicative of radical concentration and one or more image data sets indicative of radical electron relaxation times (generally $T_{1e}$, $T_{2e}$ or $T_{1e}.T_{2e}$) and manipulation of these data

sets and calibration with *ex vivo* calibration data to yield an image data set indicative of oxygen concentration. This oxygen concentration image data set can be transformed into an oxygen concentration image or can be subject to an upper or lower limit filter to identify regions of high or low oxygen concentration, which can again if desired be displayed as an image.

Broadly speaking, the Overhauser enhancement of the proton MR signal is dependent on the relaxation times $T_{1e}$ and $T_{2e}$ of the esr transition of the radical used in the OXI method. These relaxation times themselves are dependent on the concentrations of the radical and dissolved oxygen in the body fluid as well as on the temperature and chemical nature of the body fluid. However while the Overhauser enhancement can easily be used to determine the oxygen concentration for an isolated small volume sample of known radical concentration *ex vivo*, the determination of oxygen concentration *in vivo* is complicated since the Overhauser enhancement is also strongly dependent on the sample structure for a large non-isolated sample, such as a living body, due *inter alia* to non-uniform radiation penetration into the large sample.

Thus although the OXI method requires calibration data, obtained for a range of radical and oxygen concentrations in a fluid sample (e.g. blood) which corresponds to the body fluid in which oxygenation is to be determined and at a pre-set temperature (e.g. 37°C), further data manipulation is required in order to extract the *in vivo* oxygen concentrations from the OMRI signals detected for the sample.

The calibration data are generated *ex vivo* by determining Overhauser enhancement values for the radical in the selected body fluid, at the selected temperature and at a range of oxygen (and preferably also radical) concentrations. The intrinsic esr relaxation times for the radical can be determined, under the same conditions, using a conventional esr spectrometer equipped with a temperature controller, with the oxygen concentration being determined using a method known to produce accurate and reproducible results (see Ravin et al J. Appl. Physiol. 18 : 784-790(1964)).

In general, radical concentrations up to 0.2, preferably up to 1.0, especially up to 1.5mM, and oxygen concentrations of up to 0.1, preferably up to 0.5mM should be investigated to generate the calibration data.

Calibration of a blood sample at 37° can be used to determine maximum Overhauser enhancement (ie. at infinite VHF power and infinite radical concentration and $T_1$. Equations which relate $T_{1e}$ and $T_{2e}$ to the radical and oxygen concentration by a linear relationship can be derived experimentally for whatever radical is used in the method of the invention. The equations are:

$$2(\sqrt{3}\, Y_e\, T_{2e})^{-1} = x + y\, C_{rad} + z\, C_{o2} \quad (1)$$

$$2\,(\sqrt{3}\, Y_e\, T_{1e})^{-1} = a + b\, C_{rad} + c\, C_{o2} \quad (2)$$

$$(Y_e \sqrt{T_{1e}\, T_{2e}})^{-1} = h + j\, C_{rad} + k\, C_{o2} \quad (3)$$

where x, y, z, a, b, c, h, j and k (in m G) are the experimentally determinable coefficients characteristic of the chosen radical. $Y_e$ is the electron gyromagnetic ratio, $C_{rad}$ is the radical concentration (mM) , $C_{o2}$ is the oxygen concentration (mM), $T_{1e}$ and $T_{2e}$ are electron relaxation time(s).

With this calibration data, if $T_{1e}$, $T_{2e}$ or $T_{1e}.T_{2e}$ are calculated for a pixel in the sample's OMRI image then equations (1), (2) or (3) can easily be used to determine the oxygen concentration for that pixel. The radical concentration can be determined by manipulation of the MR signals detected in the OXI method (as described in WO97/09633) whereby to generate a radical concentration image data set.

Generally speaking, for use in the OXI method it is preferred for the radical to be stable under physiological conditions with a sufficiently long half life (at least one minute, preferably at least 30 minutes) and a long electronic relaxation time and good relaxivity. It will be apparent from the discussion of the OXI method that the sensitivity of the oxygen measurement will be improved with radicals having narrow linewidth esr transitions, e.g. up to 500mG, preferably less than 150mG, especially less than 60mG.

Preferably, the radical selected for use in the OXI method should distribute substantially into the extracellular fluid (i.e. should be an ECF agent) since the effects of paramagnetic iron (e.g. the iron within the red blood cells) may be avoided there.

Another preferred characteristic of the radical chosen for use in the OXI method is that they should have a low self-broadening effect, preferably less than 100mG, especially preferably between 0 and 50mG per mM of the radical itself.

The radical compounds according to the invention may be prepared from their non-radical precursor compounds by conventional radical generation methods. Suitable non-radical precursor compounds include the corresponding triaryl methanes, triaryl methyl halides and triaryl methanols, and derivatives, e.g. ethers, of the triaryl methanols.

In a further aspect the invention provides a process for the preparation of the radical compounds according to the invention which comprises subjecting a radical precursor therefor to a radical generation step and optionally subsequently modifying the substitution on the aryl moieties, e.g. by oxidation or reduction. By such modification for example sulphide substituents, (e.g. $-SCH_3$ or $-SCH_2COOEt$) may be oxidized to the corresponding sulphones so avoiding problems of acidic hydrogens prior to radical formulation. Similarly lipophilic substituents (such as $-SCH_2COOEt$) may be reduced to corresponding hydrophilic substituents (e.g. $-SCH_2CH_2OH$).

The radical-precursor conveniently contains a group displaceable to produce a radical eg. an OH, Hal, H, COOH, -CO.O.O.CO.C- or -C.NN.C- group. Methods for preparing radical compounds from these precursors are disclosed in *inter alia* WO-A-91/12024 and WO-A-96/39367.

The non-radical precursors may themselves be prepared by methods conventional in the art and a number of suitable methods are described in WO-A-91/12024 and WO-A-96/39367.

Radicals with long half lives in aqueous solution, for example at least one hour, preferably ten days, more preferably fifty days and especially preferably at least one year are particularly desirable for use in *in vivo* imaging.

For use in OMRI in general or the OXI method, the radical compounds according to the invention are physiologically tolerable or in a physiologically tolerable form (eg. in solution, encapsulated or as a precursor). Conveniently the compounds are formulated into contrast media together with conventional pharmaceutical carriers or excipients. Contrast media manufactured or used according to this invention may contain, besides the radical (or the non-radical precursor where radical formation is to be effected immediately before administration or *in situ*), formulation aids such as are conventional for therapeutic and diagnostic compositions in human or veterinary medicine. Thus the media may for example include solubilizing agents, emulsifiers, viscosity enhancers, buffers, etc. The media may be in forms suitable for parenteral (e.g. intravenous) or enteral (e.g. oral) application, for example for application directly into body cavities having external voidance ducts (such as the gastrointestinal tract, the bladder and the uterus), or for injection or infusion into the cardiovascular system. However solutions, suspensions and dispersions in physiological tolerable media will generally be preferred.

Radicals which are relatively unstable or insoluble in the sample environment may be encapsulated, e.g. in gastric juice resistant capsules containing a medium in which they are stable. Alternatively, the radical may be presented as an encapsulated freeze dried powder in a soluble capsule. Such formulations might conveniently be dissolved shortly before in vivo use.

For use in *in vivo* diagnostic imaging, the medium, which preferably will be substantially isotonic, may conveniently be administered at a concentration sufficient to yield a 1 micromolar to 10 mM concentration of the free radical in the imaging zone; however the precise concentration and dosage will of course depend upon a range of factors such as toxicity, the organ targetting ability of the contrast agent, and the administration route. The optimum concentration for the free radical represents a balance between various factors. In general, optimum concentrations would in most cases lie in the range 0.1 to 100mM, especially 0.2 to 10mM, more especially 0.5 to 5mM. Compositions for intravenous administration would preferably contain the free radical in concentrations of 10 to 1000mM especially 50 to 500 mM. For ionic materials, the concentration will particularly preferably be in the range 50 to 200mM, especially 130 to 170mM and for non-ionic materials 200 to 400mM, especially 290 to 330mM. For imaging of the urinary tract or the renal or biliary system however, compositions may perhaps be used having concentrations of for example 10 to 100mM for ionic or 20 to 200mM for non-ionic materials. Moreover for bolus injection the concentration may conveniently be 0.1 to 100mM, preferably 5 to 25mM, especially preferably 6 to 15mM.

The present invention will now be further illustrated by the following non-limiting Examples (percentages, parts and ratios are by weight and temperatures are in degrees Celsius unless otherwise stated).

Example 1

Benzo [1, 2-d:4,5-d']bis (1, 3) dithiole-2,2,6,6-tetraacetic acid ethyl ester.

The reaction was performed in dry flask under argon atmosphere. 1,2,4,5-Benzotetrathiole (74 g, 0.359 mol) and diethylacetone dicarboxylate (195.5 ml, 1.076 mol) were mixed with dichloromethane (3500 ml) and the mixture was cooled to -10°C. Fluoroboric acid (197.7 ml, 1.434 mol) was added and the cooling bath was changed to an icebath. The mixture was stirred at 0°C for 90 minutes and then poured into solid sodium carbonate (300 g) under vigorous stirring. The slurry was filtered and the filtrate was evaporated to dryness. The solid residue was triturated with heptane (2 x 250 ml) and dried in a stream of air.
Yield: 138.6 g (67 %)
$^1H$ NMR ($CDCl_3$): 6.97 (s, 2H), 4.16 (q, J=7.2 Hz, 8H), 3.49 (s, 8H) 1.26 (t, J=7.2 Hz, 12H).

Example 2

2,2,6,6-Tetra(hydroxyethyl)benzo[1,2-d:4,5-d']bis(1,3)dithiole.

A dry flask under argon atmosphere was charged with diethyl ether (2600 ml) and $LiAlH_4$ (21.2 g 0.56 mol). Benzo[1,2-d:4,5-d']bis(1,3)dithiole-2,2,6,6-tetraacetic acid ethyl ester (80.2 g, 0.139 mol) was added and the mixture was refluxed for 26 h. The mixture was cooled to ambient temperature and ethanol (165 ml) was carefully added followed by water (410 ml). The ether was decanted off and the white precipitate was stirred with water (3300 ml) to give a slurry. After acidification with hydrochloric acid the slurry was filtered and the crude product was washed with water and dried.
Yield: 55.0 g (97 %)
$^1$H NMR (DMSO-d6): 7.19 (s, 2H), 4.64 (t, J=5.7 Hz, 4H), 3.56 (q, J=5.7 Hz, 8H), 2.22 (t, J=6.3 Hz)

Example 3

2,2,6,6-Tetra(t-butoxyethyl)benzo[1,2-d:4,5-d']bis(1,3)dithiole.

A dry flask under argon was charged with 2,2,6,6-tetra(hydroxyethyl)benzo[1,2-d:4,5-d']bis(1,3)dithiole (50.3 g, 0.124 mol), tetrahydrofuran (1200 ml) and isobutene (300 ml). Trifluoromethane sulphonic acid (21.8 ml, 0.248 mol) was added over 1 minute and the mixture was stirred for 1 h. The reaction mixture was poured into solid sodium carbonate (750 g) under vigorous stirring. After filtration through a silica pad the filtrate was evaporated to dryness and the solid residue was recrystallized from ethanol to give white needles.
Yield: 61.6 g (79 %).
$^1$H NMR ($CDCl_3$): 6.95 (s, 2H), 3.56 (t, J=6.6, 8H) 2.34 (t, J=6.6 Hz, 8H) 1.18 (s, 36H)

example 4

2,2,6,6-Tetra(t-butoxyethyl)4-iodo-benzo[1,2-d:4,5-d']bis(1,3)dithiole.

A dry flask under argon was charged with 2,2,6,6-tetra(t-butoxyethyl)benzo[1,2-d:4,5-d']bis(1,3)dithiole (60.0 g, 95.08 mmol) and dry tetrahydrofuran (2000 ml). The mixture was cooled to - 20°C and n-Butyl lithium (76 ml of a 2.5 M solution in hexane, 0.19 mol) was added over 3 minutes. The mixture was stirred for 20 minutes at -20°C and then a solution of iodine (120 g, 0.475 mol) in tetrahydrofuran (500 ml) was added. The reaction mixture was poured into an aqueous sodium bisulphite solution and extracted with diethyl ether. The organic phase was washed once with an aqueous solution of sodium bisulphite and once with brine, dried ($MgSO_4$) and evaporated.
The product was purified by chromatography on silica gel using a mixture of $CH_2Cl_2$ and ethyl acetate as the eluent.
Yield: 36.7 g (51 %).
$^1$H NMR ($CDCl_3$): 6.87 (s, 1H), 3.57 (t, J=6.6 Hz, 8H), 2.35 (t, J=6.6 Hz 12 H).

Example 5

Tris(2,2,6,6-tetra-(t-butoxy-ethyl)benzo[1,2-d:4,5-d']bis(1,3)dithiole-4-yl)methanol.

A dry flask under argon was charged with 2,2,6,6-tetra(t-butoxyethyl)4-iodo-benzo[1,2-d:4,5-d']bis(1,3)dithiole (29.0 g 38.3 mmol) and dry diethyl ether (520 ml). The mixture was cooled to - 78°C and n-butyl lithium (15.3 ml of a 2.5 M solution in hexane, 38.3 mmol) was added and the cooling bath was removed. After 30 minutes an etherial solution (0.319 M) of diethyl carbonate (40 ml, 12.76 mmol) was added dropwise over 120 minutes. Ten minutes after complete addition, the mixture was poured into aqueous $NaH_2PO_4$ and extracted with diethyl ether (2x250 ml). The organic phase was washed with water, dried ($MgSO_4$) and evaporated.
The product was purified by chromatography on silica gel using a mixture of $CH_2Cl_2$ and acetonitrile as the eluent.
Yield: 15.7 g (64 %).
$^1$H NMR ($CDCl_3$) : 7.07 (s, 3H), 6.57 (s, 1H), 3.30-3.60 (m, 24H), 2.10-2.50 (m, 24H), 1.12-1.17 (m, 108H).

Example 6

Tris(8-hydroxycarbonyl-2,2,6,6-tretra-(t-butoxyethyl)benzo[1,2-d:4,5-d']bis(1,3)dithiole-4-yl)methanol.

A dry flask under argon atmosphere was charged with N,N,N',N'-tetramethylethylene diamine (12.5 ml) and

tris(2,2,6,6-tetra-(t-butoxyethyl)benzo[1,2-d:4,5-d']bis(1,3)dithiole-4-yl)methanol (960 mg, 0.5 mmol). The mixture was cooled to -20°C and n-butyl lithium (3.0 ml of a 2.5 M solution in hexane, 7.5 mmol) was added over 2 minutes. The mixture was allowed to reach ambient temperature and after 1 h the mixture was heated to 40°C and kept at this temperature for 20 minutes.
The mixture was then transferred to a dry flask containing an excess of carbon dioxide (s) and was then left to reach ambient temperature. The reaction mixture was taken up in water (200 ml) and ether (150 ml) and the phases were separated. The organic phase was extracted once more with water (100 ml) and the combined aqueous phases were acidified to pH 2 and extracted with ether (100 ml). The organic phase was dried ($MgSO_4$), evaporated and the product was purified by preparative HPLC.
Yield: 315 mg (31 %).
$^1$H NMR (DMSO): 7.03 (s, 1H), 3.18-3.58 (m, 24H); 1.95-2.30 (m, 24 H), 0.96-1.09 (m, 108H).

Example 7

Tris(8-carboxy-2,2,6,6-tetra-(hydroxyethyl)benzo[1,2-d:4,5-d']bis(1,3)dithiole-4-yl)methyl sodium salt.

To a solution of trifluoromethane sulphonic acid (8.48 ml) in acetonitrile (64.0 ml) and dichloromethane (50.0 ml) at ambient temperature was added a solution of tris(8-hydroxycarbonyl-2,2,6,6-tetra-(t-butoxyethyl)benzo[1,2-d:4,5-d']bis(1,3)dithiole-4-yl)methanol (300 mg, 0.15 mmol) in acetonitrile (8.0 ml) and dichloromethane (8.0 ml). The mixture was stirred for 7 minutes and a 26 mM solution of tin chloride (5.2 ml, 0.14 mmol) was added. After 4 minutes ice cold 1 M aqueous sodium hydroxide (96 ml) was added and the phases were separated and the aqueous phase containing the product was collected. The pH was adjusted to pH 2 and the solution was subjected to preparative HPLC. The collected fractions was evaporated to eliminate acetonitrile and the aqueous solution was poured on a pad packed with C-18 material. The pad was washed with deionized water and the product was eluted with ethanol and evaporated to dryness. To the residue was added water (5 ml) and the pH was carefully adjusted to pH 7 with diluted hydrochloric acid and the mixture was lyophilized.
Yield: 37.6 mg (18 %).
ESR (1.0 mM in $H_2O$, 100 G): singlet, linewidth 160 mG.

Example 8

Tris (8-carboxymethylthio-2,2,6,6-tetra-(2-(1-t-butoxyethyl))benzo[1,2-d:4, 5-d']bis(1,3)dithiole-4-yl)methanol

A dry flask was charged with tris(2,2,6,6-tetra-(2-(1-t-butoxyethyl))benzo[1,2-d:4,5-d'] bis(1,3)dithiole-4-yl) methanol (1.0 g, 0.521 mmol) and N,N,N',N'-tetramethyl-ethylenediamine (TMEDA, 10.4 ml). The solution was stirred at -20°C and s-BuLi (6 ml, 7.8 mmol) was added dropwise. The temperature was adjusted to -10°C, kept there for 14 minutes, and then adjusted to -25°C. Sulphur (0.58 g, 18.1 mmol) was added and the cooling bath was removed. After 90 minutes the reaction mixture was evaporated to dryness. The residue was treated with 2 M HCl (50 ml) and $CH_2Cl_2$ (40 ml). The organic phase was dried ($Na_2SO_4$) and evaporated to dryness.
Ethanol (99.5%, 40 ml), $K_2CO_3$ (4 g, 28.9 mmol), ethyl bromoacetate (1.73 g, 10.4 mmol) were added to the residue. The suspension was stirred for 2 hours, filtered and evaporated to dryness. The residue was washed with methanol/water (90/10, v/v, 20 ml) and dried. Ethanol (25 ml) and 2 M KOH (10 ml) were added, and, after stirring for one hour, the pH was adjusted to 4.5 using 2 M HCl. The precipitate was filtered off and then dissolved in diethyl ether (250 ml). Water (300 ml) was added and pH was adjusted to 10.2 using 2 M HCl. The aqueous phase was isolated, pH was adjusted to 2.5 using 2 M HCl and the product was extracted into diethyl ether (100 ml). After evaporation, 0.58 g (51%) of the product remained with a purity of 90% as ascertained by HPLC analysis.
MS (ESP, m/e): 2187.2 (M-2).

Example 9

Tris (8-carboxymethylthio-2,2,6,6-(2-(1-hydroxyethyl)) benzo[1,2-d:4,5-d']bis(1,3)dithiole-4-yl)methyl sodium salt

A flask was charged with tris(8-carboxymethylthio-2,2,6,6-tetra-(2-(1-t-butoxyethyl))benzo-[1,2-d:4,5-d'] bis (1,3)dithiole-4-yl)methanol (0.452 g, 0.244 mmol) and formic acid (25 ml). The solution was left overnight and then evaporated to dryness. The residue was dissolved in acetonitrile (17 ml) and trifluoromethanesulfonic acid (0.215 ml, 2.44 mmol) was added. After stirring for two minutes, tin(II)chloride (26.8 mg, 0.141 mmol) dissolved in acetonitrile (2.7 ml) was added. After stirring for another 1.5 minutes, the reaction mixture was poured into stirred water (500 ml). The solution was extracted with ethyl acetate (200 ml) and the organic phase was washed with water (50 ml), dried

($Na_2SO_4$) and evaporated to dryness. The product was purified by preparative HPLC (RP-18, $CH_3CN$/aqueous 0.01 M HCl 20:80). After removal of the solvents, the acid was neutralized with 1 M aqueous NaOH and the solution was then lyophilized.
Yield: 0.172 g (45 %).
MS (ESP, m/e): 1499 ($M^+$)
ESR (100 G, 2.5 mM aqueous solution): singlet, linewidth 195 mG.

Example 10

Tris (8-N,N-bis(2,2-dimethyl-1,3-dioxolane-4-methyl)aminocarbonylmethylthiol-2,2,6,6-tetra-(2-(1-t-butoxyethyl)) benzo[1,2-d:4,5-d']bis(1,3)dithiole-4-yl)methanol

A dry flask was charged with tris(2,2,6,6-tetra-(2-(1-t-butoxyethyl))benzo(1,2-d:4,5-d']bis(1,3)dithiole-4-yl) methanol (3.0 g, 1.56 mmol) and TMEDA (31.2 ml). The solution was stirred at -20°C and sec-BuLi (18 ml, 23.4 mmol) was added dropwise. The temperature was adjusted to -10°C and kept there for 14 minutes and then adjusted to -25°C. Sulphur (1.74 g, 54.4 mmol) was added and the cooling bath was removed. After 90 minutes, the reaction mixture was evaporated to dryness. The residue was treated with 2 M aqueous HCl (100 ml) and dichloromethane (100 ml). The organic phase was dried ($Na_2SO_4$) and evaporated to dryness. Ethanol (99.5%', 80 ml), $K_2CO_3$ (8 g, 57.9 mmol) and 2-bromo-N,N-bis(2,2-dimethyl-1,3-dioxolane-4-methyl)-acetamide (5.14 g, 14.0 mmol) were added to the crude intermediate. The resulting suspension was stirred for 20 hours at room temperature, filtered and evaporated to dryness. Purification was performed by preparative HPLC (RP-18, $CH_3CN/CH_2Cl_2$, 3:1).
Yield: 1.68 g (37.5%).
MS (ESP, m/e): 2869 ($M^+$).

Example 11

Tris(8-[N,N-bis(2,3-dihydroxypropyl)aminocarbonylmethylthiol-2,2,6,6-tetra-(2-(1-hydroxyethyl))benzo[1,2-d:4,5-d'] bis(1,3)dithiole-4-yl)methyl

A flask was charged with tris(8-[N,N-bis(2,2-dimethyl-1,3-dioxolane-4-methyl)aminocarbonylmethylthio]-2,2,6,6-(2-(1-t-butoxyethyl))benzo[1,2-d:4,5-d'] bis(1,3)dithiole-4-yl)methanol (0.50 g, 0,174 mmol) and dichloromethane (46 ml) at room temperature. To the stirred solution was added a solution of trifluoromethanesulfonic acid (0.62 ml) in dichloromethane (46 ml). After stirring for two minutes, a solution of tin (II) chloride (33 mg, 0.174 mmol) in acetonitrile (4.6 ml) was added. The reaction mixture was poured into water (460 ml) after another 2 minutes. The radical was purified by preparative HPLC (RP-18, $CH_3CN$/water, 5:95), neutralized with 1 M NaOH and lyophilized.
Yield: 200 mg (59 %).
MS (ESP, m/e): 1941 ($(M+1)^+$).
ESR (100 G, 1 mM in water): singlet, linewidth 274 mG.

Example 12

Benzo[1,2-d:4,5-d']bis(1,3)dithiole-2,2,6,6-tetraacetic acid methyl ester

The reaction was performed in a dry flask under argon atmosphere. 1,2,4,5-Benzotetrathiol 20.45 g, 0.097 mol) and dimethylacetone dicarboxylate (50.68 g, 0.291 mol) were mixed with dichloromethane (250 ml) and the mixture was cooled to -10°C. Fluoroboric acid etherate (53 ml, 0.388 mol) was added and the cooling bath was changed to an ice bath. The mixture was stirred at 0°C for 90 minutes and then poured into solid sodium carbonate (100 g) under vigorous stirring. The slurry was filtered and the filtrate was evaporated to dryness. The solid residue was triturated with diethyl ether (300 ml) and dried in a stream of air.
Yield: 30.4 g (60 %).
$^1H$ NMR ($CDCl_3$, 300 MHz): 6.97 (s, 2H), 3.70 (s, 12H), 3.51 (s, 8H).

Example 13

4,8-Dibromobenzo[1,2-d:4,5-d']bis(1,3)dithiole-2,2,6,6-tetraacetic acid methyl ester

A flask was charged with benzo[1,2-d:4,5-d']bis(1,3)dithiole-2,2,6,6-tetraacetic acid methyl ester (30 g, 0.058 mol) and dichloromethane (300 ml). Iodine (14.7 g, 0.0578 mol) and iodine monobromide (83.7 g, 0.405 mol) were

added and the mixture was heated to reflux for 3 hours. The mixture was cooled and poured into a stirred solution of ethanol (75 ml) and sodium bisulphite (75 g) in water (600 ml). The organic phase was separated, 100 ml water was added and the biphasic mixture was evaporated *in vacuo* to remove dichloromethane. The resulting aqueous slurry was filtered and the filter cake was washed with water (3x200 ml) and recrystallized from n-butanol (450 ml). Yield: 31.8 g (81 %).
$^1$H NMR ($CDCl_3$, 300 MHz) : 3.72 (s, 12H), 3.55 (s, 8H).

Example 14

4,8-Dibromobenzo[1,2-d :4.5-d']bis(1,3)dithiole-2,2,6,6-tetraacetic-2-$d_2$-acid methyl ester

A dry flask was charged with THF (120 ml) and methanol-$d_1$ (200 ml). Sodium (0.64 g, 0.0278 mol) was dissolved in the mixture and 4,8-dibromobenzo[1,2-d:4,5-d'] bis(1,3)dithiole-2,2,6,6-tetraacetic acid methyl ester (40.0 g, 0.0546 mol) was added. The mixture was heated to reflux for 12 hours and then evaporated in vacuo. The residue was suspended in aqueous 0.1 M $NaH_2PO_4$ (500 ml) and the slurry was filtered and washed with aqueous 0.1 M $NaH_2PO_4$ (2x50 ml). The product was dried in a stream of air.
Yield: 31.1 g (83 %).
$^1$H NMR ($CDCl_3$, 300 MHz): 3.72 (s, 12H).

Example 15

2,2,6,6-Tetra(2-(1-hydroxy-2,2-$d_2$-ethyl))-4,8-dibromobenzo[1,2-d:4,5-d']bis(1,3)dithiole

A flask under an argon atmosphere fitted with a reflux condenser was charged with THF (210 ml) and lithium borohydride (3.22 g, 0.15 mol). Methanol (5.99 ml, 0.15 mol) was added dropwise while stirring and 15 minutes after the complete addition 4,8-dibromobenzo[1,2-d:4,5-d'] bis(1,3)dithiole-2,2,6,6-tetraacetic-2-$d_2$-acid methyl ester (20 g, 0.0296 mol) was added in portions under 15 minutes. The mixture was then stirred for 2 hours and then heated to reflux for an additional 2 hours. After cooling to ambient temperature, the reaction was quenched by the dropwise addition of 20% aqueous acetic acid (75 ml). The mixture was evaporated in vacuo to remove THF and the pH was adjusted to 3 using 3 M hydrochloric acid. The obtained slurry was filtered and the filtercake was washed with water (3x100 ml) and dried in a stream of air.
Yield: 16.4 g, (98 %)
$^1$H NMR ($CDCl_3$, 300 MHz) : 4.71 (broad s, 4H), 3.57 (s, 8H).

Example 16

2,2,6,6-Tetra(2-(1-hydroxy-2,2-$d_2$-ethyl))-4-bromo-benzo[1,2-d:4,5-d']bis(1,3)dithiole

A flask was charged with dimethyl formamide (290 ml) and 2,2,6,6-tetra(2-(1-hydroxy-2,2-$d_2$-ethyl))-4,8-dibromobenzo[1,2-d:4,5-d']bis(1,3)dithiole (32 g, 0.056 mol). The starting material was dissolved with gentle heating and the solution was cooled to ambient temperature. Zn (7.5 g, 0.115 mol), $CuSO_4 \cdot 5\,H_2O$ (5.7 g, 0.023 mol) and ammonium formate (39.7 g, 0.629 mol) were added to the vigorously stirred reaction mixture. After 50 minutes the mixture was filtered and the filtrate was poured into water (1500 ml) and the pH was adjusted to 4 using 3 M hydrochloric acid. The precipitate was filtered off and washed with water (2 x 300 ml) and diethyl ether (2 x 60 ml). The crude material was recrystallized from a mixture of ethanol and water (1:1) and dried in vacuo. Yield: 18.1 g, (70 %)
$^1$H NMR ($CDCl_3$, 300 MHz): 7.18 (s, 1H), 4.66 (t, J = 5.1 Hz, 4H) 3.55 (d, J = 5.1 Hz, 8H).

Example 17

2,2,6,6-Tetra(2-(1-t-butoxy-2,2-$d_2$-ethyl))-4-bromobenzo[1,2-d:4,5-d']bis(1,3)dithiole

A dry flask under argon was charged with 2,2,6,6-tetra(2-(1-hydroxy-2,2-$d_2$-ethyl))-4-bromobenzo[1,2-d:4,5-d']bis(1,3)dithiole (31.0 g, 0.063 mol), THF (300 ml) and the headspace was flushed with isobutene. Trifluoromethane sulphonic acid (3.0 ml, 0.0349 mol) was added to the stirred mixture and a slight pressure of isobutene was applied to the flask. The mixture was stirred for 4.5 hours and then poured into a mixture of aqueous 1 M NaOH (40 ml) and water (300 ml). The phases were separated and the organic phase was diluted with diethyl ether (150 ml) and was washed with water (3 x 200 ml). The organic phase was dried ($MgSO_4$) and evaporated and the crude product was dissolved in heptane (100 ml) and poured on a silica pad (300 g). The pad was eluted with a mixture of heptane and

ether (4:1, v/v) and the eluate was evaporated to yield a white waxlike solid.
Yield: 33.8 g, (75 %).
$^1H$ NMR ($CDCl_3$, 300 MHz): 6.85 (s, 1H), 3.56 (s, 8H), 1.18 (s, 36H).

Example 18

Tris(2,2,6,6-tetra (2-(1-t-butoxy-2,2-$d_2$-ethyl))benzo[1,2-d:4,5-d']bis(1,3)dithiole-4-yl)methanol

A dry flask under argon was charged with 2,2,6,6-tetra(2-(1-butoxy-2,2-$d_2$-ethyl))-4-bromo-benzo[1,2-d:4,5-d']bis(1,3)dithiole (10.0 g, 0.0139 mol) and dry diethyl ether (140 ml). The mixture was cooled to -20°C and n-butyl lithium (5.6 ml of a 2.5 M solution in hexane, 0.014 mol) was added and the cooling bath was removed. After 30 minutes, a solution of diethyl carbonate (546 mg, 4.63 mmol) in diethyl ether was added dropwise over 2 hours. After complete addition the mixture was stirred for an additional 10 minutes and then poured into an aqueous solution of $NaH_2PO_4$ (125 ml, 0.2 M). The phases were separated and the organic phase was washed twice with brine and dried over $MgSO_4$. The organic phase was evaporated and the red oily residue was dissolved in ethanol (56 ml) and left over night. The following day the yellow crystalline product was collected and dried *in vacuo.*
Yield: 5.54 g, (62 %).
$^1H$ NMR ($CDCl_3$, 300 MHz): 7.07 (s, 3H), 6.57 (s, 1H), 3.60-3.30 (m, 24H), 1.18-1.04 (m, 108H).

Example 19

Tris(8-carboxyl-2,2,6,6-tetra(2-(1-t-butoxy-2,2-$d_2$-ethyl))-benzo[1,2-d:4,5-d']bis(1,3)dithiole-4-yl)methanol

A dry flask under argon was charged with TMEDA (89 ml) and tris(2,2,6,6-tetra(2-(1-t-butoxy-2,2-$d_2$-ethyl))-benzo[1,2-d:4,5-d']bis(1,3)dithiole-4-yl)methanol (8.65 g, 4.45 mmol). The mixture was cooled to -20°C and sec-BuLi (51.5 ml, 0.067 mol, 1.3 M in cyclohexane) was added over 4 minutes while the temperature was kept below -10°C. After complete addition the reaction mixture was stirred at -10°C for 25 minutes and then transferred onto solid $CO_2$ and left to attain ambient temperature. The thick mixture was taken up in diethyl ether (445 ml) and water (445 ml) and acidified with 6 M hydrochloric acid to pH 1.5. The phases were separated and the aqueous phase was extracted once more with diethyl ether (445 ml). The combined organic phase were dried ($MgSO_4$) and evaporated to dryness to give a yellow foam.
Yield: 5.80 g (63 %).
$^1H$ NMR ($CD_3CN$, 300 MHz): 7.18 (s, 1H), 3.60 - 3.25 (m, 24H), 1.15 - 1.02 (m, 108H).

Example 20

Tris(8-carboxyl-2,2,6,6-tetra(2-(1-hydroxy-2,2-$d_2$-ethyl))benzo[1,2-d:4,5-d']bis(1,3)dithiole-4-yl)methyl

A flask was charged with tris(8-carboxy-2,2,6,6-tetra(2-(1-t-butoxy-2,2-$d_2$-ethyl))-benzo[1,2-d:4,5-d']bis(1,3) dithiole-4-yl)methanol (5.80 g, 0.0028 mol) and formic acid (60 ml) and the mixture was stirred over night. The mixture was evaporated to dryness, the residue was dissolved in acetonitrile (30 ml) and again evaporated to dryness. The residue was dissolved in acetonitrile (12 ml) and a solution of trifluoromethane sulphonic acid (4.87 ml, 0.0558 mol) in acetonitrile (22.5 ml) was added while stirring. After 6 minutes, a solution of $SnCl_2$ (0.529 g, 0.00279 mol) in THF (2.87 ml) was added to the stirred reaction mixture. After stirring for 2 minutes, ethyl acetate (29 ml) was added directly followed by an addition of aqueous $NaH_2PO_4$ (140 ml, 2 M). The biphasic mixture was stirred vigorously for 5 minutes and the phases were separated. The organic phase was evaporated and treated with aqueous sodium hydroxide (55.8 ml, 1 M). After 15 minutes the aqueous phase was added dropwise into hydrochloric acid (112 ml 1.5 M). After 15 minutes the dark precipitate was collected and washed with diluted hydrochloric acid (2 x 10 ml, 0.1 M) followed by diethyl ether (10 ml).
Yield: 3.56 g, (92 %).

Example 21

Tris (8-carboxyl-2,2,6,6-tetra (2-(1-hydroxy-2.2-$d_2$-ethyl))-benzo[1,2-d:4,5-d']bis(1,3)dithiole -4-yl)methyl sodium salt

To a sample of the solid radical triacid, obtained in Example 20, water was added and the solution was neutralized to pH 7 using 0.05 M sodium hydroxide and the solution was lyophilized.
ESR (1.0 mM in $H_2O$, 100 G): singlet, linewidth 70 mG.

Example 22

N,N-Bis(2,2-dimethyl-1,3-dioxolane-4-methyl)-2-bromo- acetamide

A flask was charged with N,N-bis(2,3-dihydroxyprop-1-yl)-amine (30.0 g, 0.1816 mol), 2,2-dimethoxypropane (120 ml), dimethyl formamide (100 ml) and concentrated sulphuric acid (20 ml). The mixture was stirred overnight and then poured into a mixture of saturated $NaHCO_3$ (500 ml) and sodium hydroxide (16. 0 g, 0.4 mol) while stirring. The aqueous phase was extracted with diethyl ether (3 x 200 ml) and the organic phase was dried ($MgSO_4$) and evaporated to give a pale yellow oil. The oil was dissolved in a mixture of dichloromethane (75 ml) and 0.03 M aqueous sodium hydroxide (2000 ml). A solution of bromoacetyl bromide (11.81 g, 0.0585 mol) in dichloromethane (75 ml) was added dropwise over 20 minutes and the pH of the reaction mixture was continuously monitored during the addition and was kept close to 7 by addition of 1 M aqueous sodium hydroxide. After complete addition the organic phase was separated and washed with water (2 x 150 ml), dried ($MgSO_4$) and evaporated to give a pale yellow oil.
Yield: 30.7 g (79 %).
$^1H$ NMR ($CDCl_3$, 300 MHz): 4.35-4.25 (m, 2H), 4.16-4.02 (m, 3H), 3.93-3.85 (m, 2H), 3.75-3.55 (m, 4H), 3.25-3.17 (m, 1H), 1.42-1.40 (m, 6H), 1.33-1.31 (m, 6H).

Example 23

Tris(8-carboxyl-2,2,6,6-tetra(hydroxyethyl)-benzo[1,2-d:4,5-d']bis(1,3)dithiole-4-yl)methyl N,N-bis(1,2-dihydroxyprop-3-yl)-acetamid-2-yl-ester

A flask was charged with dimethyl formamide (600 ml) and tris(8-carboxyl-2,2,6,6-tetra(hydroxyethyl)-benzo [1,2-d:4,5-d']bis(1,3)dithiole-4-yl)methyl sodium salt (2.57 g, 1.8 mmol). N,N-bis(2,2-dimethyl-1,3-dioxolane-4-methyl)-2-bromo-acetamide (6.60 g, 18 mmol): was added followed by a second portion (6.60 g, 18 mmol) after 15 minutes. The mixture was stirred for 60 minutes and the solvent was removed by evaporation. The residue was dissolved in a mixture of acetonitrile (100 ml) and water (150 ml) and 2 M hydrochloric acid (10 ml) was added. After 2 hours the acetonitrile was removed by evaporation and the product was purified by preparative HPLC.
Yield: 2.70 g (76 %).
ESR (1.0 mM in $H_2O$, 100 G): singlet, linewidth 226 mG.

## Claims

**1.** A radical compound of formula I

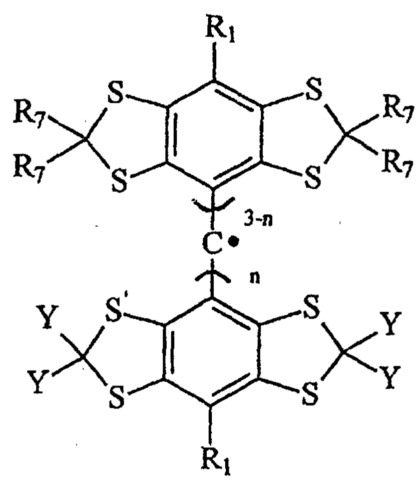

(I)

(wherein:

each $R^1$ which may be the same or different represents a hydrogen atom or group of formula -M or -XM;
each X which may be the same or different represents an oxygen or sulphur atom or a group CO or $S(O)_m$

(where m is 1 to 3);

M represents a water solubilising group;

each $R^7$ which may be the same or different represents a hydrogen atom, or a hydrocarbon group, or a water solubilising group M or two groups $R^7$ together with the atom to which they are bound represent a carbonyl group or a 5 to 8 membered cycloalkylidene, mono- or di-oxacycloalkylidene, mono- or di-azacycloalkylidene or mono- or di-thiacycloalkylidene group and $R^7$ where it is other than hydrogen, is optionally substituted by a hydroxyl group, an optionally alkoxylated, optionally hydroxylated acyloxy or alkyl group or a water solubilising group M;

n denotes 1, 2 or 3; and

each group Y denotes $CH_2CH_2OR_{22}$ wherein $R_{22}$ is H or $C_{1-6}$-alkyl or a deuterated analog thereof)

or a deuterated analog, precursor or salt thereof.

**2.** A radical compound as claimed in claim 1 where in $R_{22}$ is hydrogen or t-Bu.

**3.** A radical compound as claimed in any preceding claim wherein n denotes 3.

**4.** A radical compound as claimed in claim 1 being

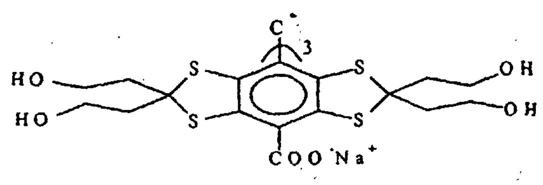

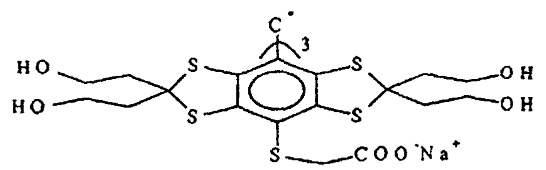

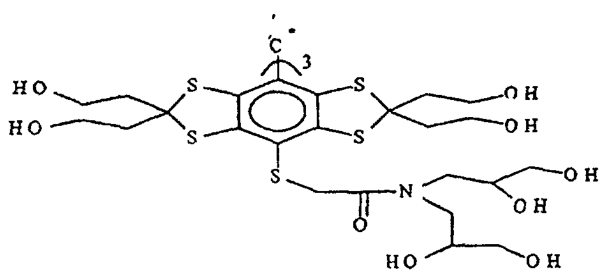

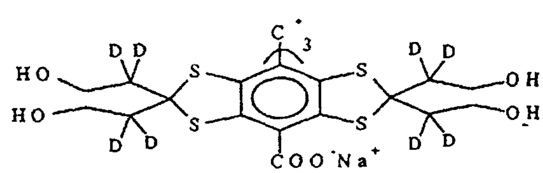

or

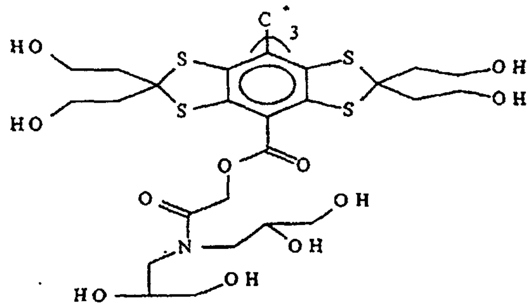

5. A magnetic resonance imaging contrast medium comprising a radical compound as claimed in any of claims 1 to 4 together with at least one pharmacologically acceptable carrier or excipient.

6. A method of magnetic resonance investigation of a sample, said method comprising introducing into said sample a radical compound as claimed in any of claims 1 to 4, exposing said sample to a first radiation of a frequency selected to excite electron spin transitions in said radical, exposing said sample to a second radiation of a frequency selected to excite nuclear spin transitions in selected nuclei in said sample, detecting free induction decay signals from said sample, and, optionally, generating an image or dynamic flow data from said detected signals.

7. A method for determining oxygen concentration in a sample, said method comprising the following steps:

introducing into said sample an effective amount of a physiologically tolerable radical compound as claimed in any of claims 1 to 4;
irradiating said sample with radiation of an amplitude and frequency selected to stimulate an electron spin resonance transition of said radical;
detecting electron spin resonance enhanced magnetic resonance signals from said sample under at least first, second and third conditions, whereby under said first and second conditions said radiation is of a first frequency, under said third conditions said radiation is of a second frequency different from said first frequency, under said first, second and third conditions said radiation is of a first, second and third amplitude, said first and second amplitudes at least being different from each other; and
manipulating said detected signals whereby to determine oxygen concentration in said sample.

8. A method as claimed in claim 7 comprising:

(a) introducing a radical compound as claimed in claim 1 into body tissue or the vasculature;
(b) generating a first OMRI image of said sample at VHF power $P_A$, irradiation period $T_{VHF1}$ and on resonance;
(c) generating a second OMRI image of said sample at a second VHF power $P_B$, irradiation time $T_{VHF1}$ and on-resonance;
(d) generating a third OMRI image of said sample at VHF power $P_c$, irradiation time $T_{VHF1}$ and off-resonance;
(e) manipulating the images obtained in steps (b) to (d) and calibrating using parameters determined *ex vivo* to provide an oxygen image of said sample.

9. A method as claimed in claim 8 wherein a fourth and fifth image are generated in the imaging sequence.

10. A process for preparing a radical compound as claimed in any of claims 1 to 4 comprising subjecting a radical precursor therefor to a radical generation step and optionally subsequently modifying the substitution on the aryl moieties.

11. Use of a radical compound as claimed in any of claims 1 to 4 in OMRI.

12. Use of a radical compound as claimed in any of claims 1 to 4 in oximetry.

**Patentansprüche**

1. Radikalverbindung der Formel (I)

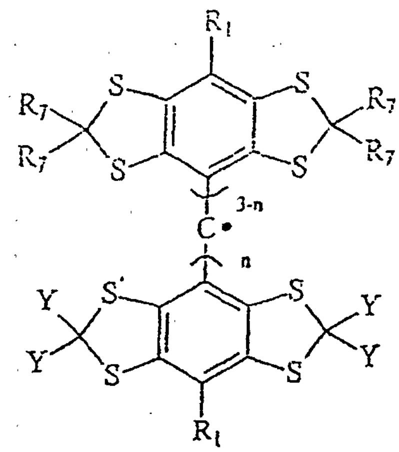

(I)

(worin:

jedes $R^1$, welche gleich oder verschieden sein können, ein Wasserstoffatom oder eine Gruppe der Formel -M oder -XM bedeutet;
Jedes X, welche gleich oder verschieden sein können, ein Sauerstoff- oder Schwefelatom oder eine Gruppe CO oder $S(O)_m$ bedeutet (worin m 1 bis 3 ist);
M eine wassersolubilisierende Gruppe bedeutet;
jedes $R^7$, welche gleich oder verschieden sein können, ein Wasserstoffatom oder eine Kohlenwasserstoffgruppe oder eine wassersolubilisierende Gruppe M bedeutet oder zwei Gruppen $R^7$ zusammen mit dem Atom, an welches sie gebunden sind, eine Carbonylgruppe oder eine 5- bis 8-gliedrige Cycloalkyliden-, Monooder Dioxacycloalkyliden-, Mono- oder Diazacycloalkyliden- oder Mono- oder Dithiacycloalkylidengruppe bedeuten und $R^7$, wenn es von Wasserstoff verschieden ist. wahlweise substituiert ist durch eine Hydroxylgruppe, eine wahlweise alkoxylierte, wahlweise hydroxylierte Acyloxy- oder Alkylgruppe oder eine wassersolubilisierende Gruppe M;
n die Bedeutung 1, 2 oder 3 hat; und
jede Gruppe Y die Bedeutung $CH_2CH_2OR_{22}$ hat, worin $R_{22}$ H oder $C_{1-6}$-Alkyl bedeutet oder ein deuteriertes Analogon hiervon)

oder ein deuteriertes Analogon, Vorläufer oder Salz hiervon.

**2.** Radikalverbindung nach Anspruch 1, wobei $R_{22}$ Wasserstoff oder t-Bu ist.

**3.** Radikalverbindung nach mindestens einem vorangehenden Anspruch. wobei n die Bedeutung 3 hat.

**4.** Radikalverbindung nach Anspruch 1, nämlich

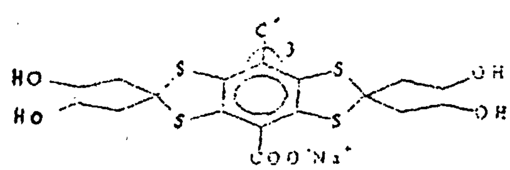

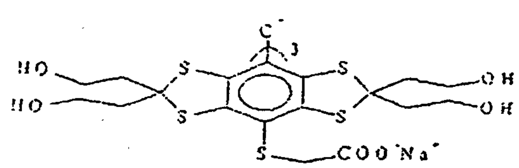

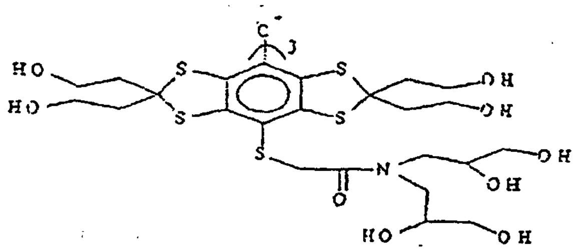

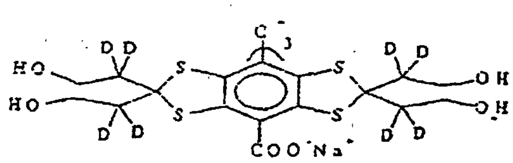

oder

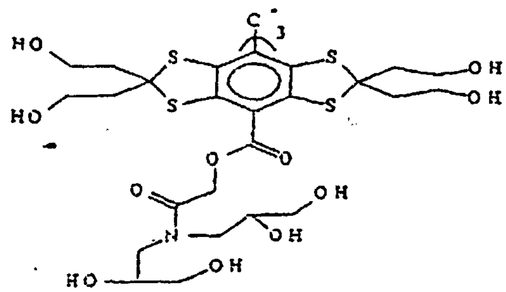

**5.** Kontrastmedium für die Bilderzeugung durch magnetische Resonanz, umfassend eine Radikalverbindung nach mindestens einem der Ansprüche 1 bis 4, zusammen mit mindestens einem pharmakologisch annehmbaren Träger oder Exzipienten.

**6.** Verfahren zur Untersuchung einer Probe mittels magnetischer Resonanz, wobei das Verfahren das Einbringen einer Radikalverbindung nach mindestens einem der Ansprüche 1 bis 4 in die Probe, Aussetzen der Probe einer ersten Strahlung einer Frequenz, so gewählt, um Elektronenspinübergänge in dem Radikal anzuregen, Aussetzen der Probe einer zweiten Strahlung einer Frequenz, so gewählt, um Kernspinübergänge in ausgewählten Kernen in der Probe anzuregen, Detektieren freier Induktionsabfallsignale aus der Probe und, wahlweise Erzeugen eines Bildes oder dynamischer Strömungsdaten aus den detektierten Signalen umfasst.

**7.** Verfahren zur Bestimmung der Sauerstoffkonzentration in einer Probe, wobei das Verfahren die folgenden Schritte umfasst:

Einbringen einer wirksamen Menge einer physiologisch tolerierbaren Radikalverbindung nach mindestens einem der Ansprüche 1 bis 4 in die Probe;
Bestrahlen der Probe mit Strahlung einer Amplitude und Frequenz, so gewählt, um einen Elektronenspinresonanzübergang des Radikals zu stimulieren;
Detektieren Elektronenspinresonanz-verstärkter, magnetischer Resonanzsignale aus der Probe unter mindestens ersten, zweiten und dritten Bedingungen, wobei unter den ersten und zweiten Bedingungen die Strahlung eine erste Frequenz besitzt, unter den dritten Bedingungen die Strahlung eine von der ersten Frequenz verschiedene, zweite Frequenz besitzt, unter den ersten, zweiten und dritten Bedingungen die Strahlung eine erste, zweite und dritte Amplitude besitzt, wobei die ersten und zweiten Amplituden zumindest voneinander verschieden sind; und
Manipulieren der detektierten Signale, um hierdurch die Sauerstoffkonzentration in der Probe zu bestimmen.

**8.** Verfahren nach Anspruch 7, umfassend:

(a) Einbringen einer Radikalverbindung nach Anspruch 1 in Körpergewebe oder in die Vaskulatur;

(b) Erzeugen eines ersten OMRI-Bildes der Probe bei VHF-Leistung $P_B$. Bestrahlungszeit $T_{VHF1}$ und An-Resonanz;

(c) Erzeugen eines zweiten OMRI-Bildes der Probe bei einer zweiten VHF-Leistung $P_B$, Bestrahlungszeit $T_{VHF1}$ und An-Resonanz;

(d) Erzeugen eines dritten OMRI-Bildes der Probe bei VHF-Leistung PC. Bestrahlungszeit $T_{VHF1}$ und Aus-Resonanz:

(e) Manipulieren der in den Schritten (b) bis (d) erhaltenen Bilder und Kalibrieren unter Verwendung von ex vivo bestimmten Parametern, um ein Sauerstoffbild der Probe vorzusehen.

**9.** Verfahren nach Anspruch 8, wobei ein viertes und ein fünftes Bild bei der Bilderzeugungssequenz erzeugt werden.

**10.** Verfahren zur Herstellung einer Radikalverbindung nach mindestens einem der Ansprüche 1 bis 4, umfassend das Unterziehen eines Radikalvorläufers hiervon einem Radikalerzeugungsschritt und wahlweise nachfolgend Modifizieren der Substitution an den Aryleinheiten.

**11.** Verwendung einer Radikalverbindung nach mindestens einem der Ansprüche 1 bis 4 in OMRI.

**12.** Verwendung einer Radikalverbindung nach mindestens einem der Ansprüche 1 bis 4 in der Oximetrie.

**Revendications**

**1.** Composé radicalaire de formule I

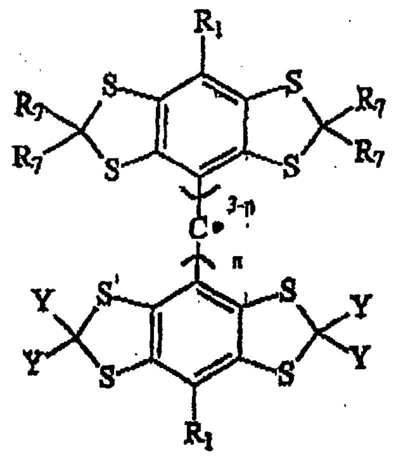

(I)

(dans laquelle :

chaque $R^1$ qui peut être identique ou différent représente un atome d'hydrogène ou un groupe de formule -M ou -XM;

chaque X qui peut être identique ou différent représente un atome d'oxygène ou de soufre ou un groupe CO ou $S(O)_m$ (où m est de 1 à 3);

M représente un groupe solubilisant dans l'eau ;

chaque $R^7$ qui peut être identique ou différent représente un atome d'hydrogène, ou un groupe hydrocarboné, ou un groupe solubilisant dans l'eau M ou deux groupes $R^7$ représentent ensemble avec l'atome auquel ils sont liés un groupe carbonyle ou un groupe cycloalkylidène, mono- ou dioxacycloalkylidène, mono- ou diazacycloalkylidène ou mono- ou dithiacyclo-alkylidène à 5 à 8 chaînons et $R^7$ lorsqu'il est différent de l'hydrogène, est éventuellement substitué par un groupe hydroxyle, un groupe acyloxy ou alkyle éventuellement alcoxylé, éventuellement hydroxylé ou un groupe solubilisant dans l'eau M ;

n représente 1,2 ou 3 ; et

chaque groupe Y représente $CH_2CH_2OR_{22}$, $R_{22}$ étant H ou alkyle en $C_1$ à $C_6$ ou un analogue deutéré de celui-ci)

ou un analogue deutéré, un précurseur ou un sel de celui-ci.

2. Composé radicalaire selon la revendication 1 dans lequel $R_{22}$ est l'hydrogène ou t-Bu.

3. Composé radicalaire selon l'une quelconque des revendications précédentes dans lequel n représente 3.

4. Composé radicalaire selon la revendication 1 étant

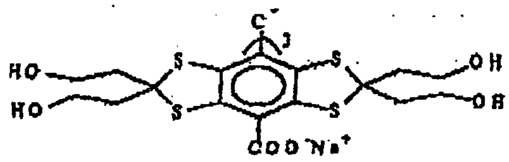

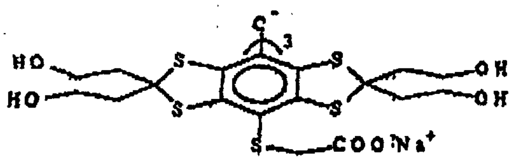

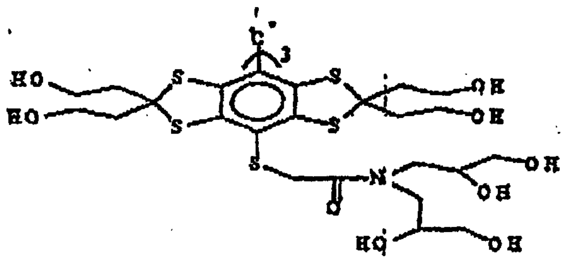

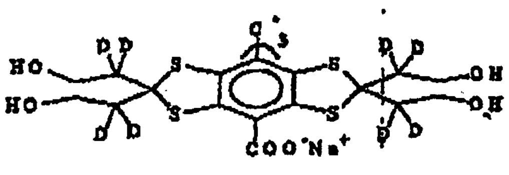

ou

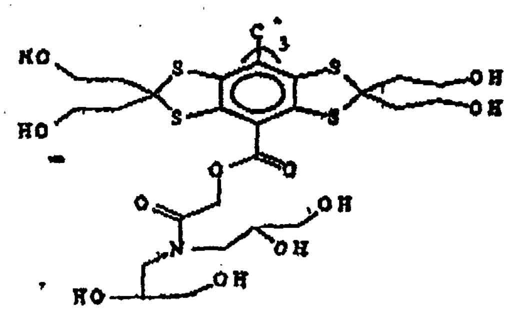

5. Agent de contraste pour imagerie par résonance magnétique comprenant un composé radicalaire selon l'une quelconque des revendications 1 à 4 en même temps qu'au moins un support ou excipient pharmacologiquement acceptable.

6. Procédé d'analyse d'un échantillon par résonance magnétique, ledit procédé comprenant l'introduction dans ledit échantillon d'un composé radicalaire selon l'une quelconque des revendications 1 à 4, l'exposition dudit échantillon à un premier rayonnement d'une fréquence choisie pour exciter les transitions de spin électronique dans ledit radical, l'exposition dudit échantillon à un second rayonnement à une fréquence choisie pour exciter les transitions de spin nucléaire dans des noyaux choisis dans ledit échantillon, la détection de signaux de décroissance d'induction libre, et, éventuellement, la génération d'une image ou de données d'écoulement dynamique à partir desdits signaux détectés.

7. Procédé de détermination de la concentration en oxygène dans un échantillon, ledit procédé comprenant les étapes suivantes :

   introduire dans ledit échantillon une quantité efficace d'un composé radicalaire physiologiquement tolérable selon l'une quelconque des revendications 1 à 4;
   irradier ledit échantillon avec un rayonnement d'une amplitude et d'une fréquence choisies pour stimuler une transition de résonance de spin électronique dudit radical ; détecter les signaux de résonance magnétique renforcés par la résonance de spin électronique provenant dudit échantillon dans au moins les première, seconde et troisièmes conditions, ce par quoi dans lesdites première et seconde conditions ledit rayonnement est d'une première fréquence, dans lesdites troisièmes conditions ledit rayonnement est d'une seconde fréquence différente de ladite première fréquence, dans lesdites première, seconde et troisième conditions ledit rayonnement est d'une première, seconde et troisième amplitude, lesdites première et seconde amplitudes étant au moins différentes l'une de l'autre; et
   manipuler lesdits signaux détectés pour déterminer ainsi la concentration d'oxygène dans ledit échantillon.

8. Procédé tel que revendiqué à la revendication 7 comprenant le fait de:

   (a) introduire un composé radicalaire selon la revendication 1 dans un tissu corporel ou le système vasculaire;
   (b) générer une première image OMRI dudit échantillon à une puissance VHF $P_A$, une période d'irradiation $T_{VHF1}$ et en résonance;
   (c) générer une seconde image OMRI dudit échantillon à une seconde puissance VHF $P_B$, un temps d'irradiation $T_{VHF1}$ et en résonance;
   (d) générer une troisième image OMRI dudit échantillon à une puissance VHF $P_C$, un temps d'irradiation $T_{VHF1}$ et hors résonance ;
   (e) manipuler les images obtenues dans les étapes (b) à (d) et calibrer en utilisant des paramètres déterminés *ex vivo* pour obtenir une image d'oxygène dudit échantillon.

9. Procédé selon la revendication 8 dans lequel une quatrième et cinquième images sont générées dans la séquence d'imagerie.

10. Procédé de préparation d'un composé radicalaire selon l'une quelconque des revendications 1 à 4 comprenant la soumission d'un précurseur radicalaire correspondant à une étape de génération de radical et en modifiant éventuellement la substitution sur les fragments aryle.

11. Utilisation d'un composé radicalaire selon l'une quelconque des revendications 1 à 4 en OMRI.

12. Utilisation d'un composé radicalaire selon l'une quelconque des revendications 1 à 4 en oxymétrie.